# EUROPÄISCHE PATENTSCHRIFT

(11) **EP 3 328 606 B1**
(45) Veröffentlichungstag und Bekanntmachung des Hinweises auf die Patenterteilung: **02.11.2022**
(21) Anmeldenummer: 16753596.2
(22) Anmeldetag: 27.07.2016
(51) Int. Cl.: B29C 35/04, B29C 37/02, B29C 71/02, A61F 2/30

(54) **VERFAHREN ZUR BEARBEITUNG EINES POLYMERWERKSTÜCKS FÜR EINEN EINSATZ IN EINEM GELENKIMPLANTAT**
METHOD FOR TREATING A POLYMER WORKPIECE FOR USE IN A JOINT IMPLANT
PROCÉDÉ DE TRAITEMENT D'UNE PIÈCE EN POLYMÈRE DESTINÉE À ÊTRE UTILISÉE DANS UN IMPLANT ARTICULAIRE

(30) Priorität: 31.07.2015 DE 102015214668
(43) Veröffentlichungstag der Anmeldung: 06.06.2018
(73) Patentinhaber: Waldemar Link GmbH & Co. KG, 22339 Hamburg (DE)
(72) Erfinder: LINK, Helmut D., 22397 Hamburg (DE); SCHÖTTLER, Carsten, 22415 Hamburg (DE)
(74) Vertreter: AWA Denmark A/S
(86) Internationale Anmeldenummer: PCT/EP2016/067890
(87) Internationale Veröffentlichungsnummer: WO 2017/021249

(56) Entgegenhaltungen:
- WO-A1-2014/126908
- DE-A1-102012 110 819
- DE-C1- 19 743 076
- DE-U1- 8 609 000
- US-A1- 2009 030 524

## Beschreibung

### TECHNISCHES GEBIET

Die vorliegende Erfindung betrifft ein Verfahren zur Bearbeitung eines Polymerwerkstücks, das für den Einsatz in einem Gelenkimplantat vorgesehen ist, sowie eine mit diesem Verfahren hergestellte Komponente eines Gelenkersatzes oder Instrumentariums für eine Implantation eines Gelenkersatzes.

### STAND DER TECHNIK

Polymerwerkstoffe werden bei Implantaten in der Orthopädie auf vielseitige Weise eingesetzt. So dienen sie bei Gelenkimplantaten, sogenannten Endoprothesen, häufig als Werkstoff, mit dem die künstliche Gelenkfläche ausgebildet wird. Die Verankerung des Polymers im Knochengewebe erfolgt mithilfe einer Verankerungskomponente, die beispielsweise aus einer Stahllegierung oder einer Titanlegierung hergestellt ist. Bei der Verankerung der Metallkomponente wächst anliegendes Knochengewebe nach der Implantation in die Prothese ein oder sie wird mit Hilfe von Knochenzement im Knochengewebe fixiert. Folglich ist die Gelenkkomponente aus dem Polymerwerkstoff bevorzugt als Einsatz oder Vorsatz ausgeführt, der an der Verankerungskomponente befestigt wird.

Ein in der Endoprothetik häufig eingesetzter Polymerwerkstoff ist Polyethylen (PE) und insbesondere UHMWPE (Ultra High Molecular Weight Polyethylene). Dessen Oberfläche zeichnet sich im Vergleich zu reinen Metallpaarungen durch einen niedrigeren Reibkoeffizienten und eine hohe Anpassungsfähigkeit aus. Letztere führt zu einem geringeren Abrieb insbesondere während der Einlaufphase des Implantats.

Nichtsdestotrotz kommt es auch bei den aus Polyethylen hergestellten Gelenkkomponenten zunächst vor allem aufgrund von Fertigungsungenauigkeiten und später aufgrund der täglichen Belastung zu Abrieb. Diesbezüglich ist seit Längerem bekannt, dass die sich während des Abriebs lösenden Polymerpartikel eine Osteolyse (lat.: Knochenauflösung) hervorrufen können (Willert, H.G.; Buchhorn, G.H.; Hess, T.: "Die Bedeutung von Abrieb und Materialermüdung bei der Prothesenlockerung an der Hüfte", Orthopäde 18: 350-369, 1989). Bei einer Osteolyse gelangen von der Polymerkomponente abgelösten Polymerpartikel in die Umgebung des Knochengewebes, wodurch eine Fremdkörperreaktion ausgelöst werden kann. Körpereigene Zellen umgeben dann die Polymerpartikel in Form von Zellumwucherungen. Als Folge hiervon kann das Knochengewebe verdrängt werden, sodass es mit erhöhter Wahrscheinlichkeit zu einer Lockerung der Endoprothese kommen kann. Gerade in jüngster Vergangenheit wurde der Entstehung dieser Gewebewucherungen erhöhte Aufmerksamkeit geschenkt, was sich auch in der Bezeichnung dieser nicht krebsartigen Wucherung als Pseudotumor wirkungsvoll niederschlägt.

Hiervon ausgehend ist es von Interesse, die Anzahl von Polymerpartikeln bei einer Endoprothese möglichst gering zu halten, um Zellumwucherungen und einer damit einhergehenden Lockerung der Endoprothese vorzubeugen.

Die US 2009/0030524 A1 offenbart ein Verfahren zur Bereitstellung eines nicht eluierenden, mit einem Antioxidans versetzten UHMWPE in Form einer Gelenkimplantatkomponente, wobei das Verfahren die folgenden Schritte umfasst: (a) Bereitstellen eines Rohlings; (b) Bestrahlen des Rohlings mit γ-Bestrahlung, um das UHMWPE zu vernetzen; (c) Versetzen der vernetzten Vorform, indem sie einer Antioxidanszusammensetzung bei einer Temperatur unterhalb des Schmelzpunkts des UHMWPE ausgesetzt wird; (d) Entfernen des versetzten Materials von dem Kontakt mit der Antioxidanszusammensetzung; und dann (e) Tempern durch Erhitzen des dotierten Materials auf eine Temperatur über 30°C und unter dem Schmelzpunkt des UHMWPE; gefolgt von (f) Herstellen einer Gelenkimplantatkomponente aus dem versetzten Material, wobei mindestens 1 mm, aber nicht mehr als etwa 15 mm Material entfernt werden, um diese Komponente herzustellen.

DE 86 09 000 U1 offenbart eine Vorrichtung zur Behandlung von Werkstücken mit einem brennbaren Gasgemisch, wobei beim thermischen Entgraten von Werkstücken ein ungezielter Abtrag erfolgt, bei dem das gesamte Werkstück einem Hitzeschock ausgesetzt wird, wobei die erfindungsgemäße Vorrichtung den Vorteil hat, dass eine für hohe Drücke ausgelegte Entgratkammer mit aufwendigem Schließsystem und entsprechend ausgelegtem Maschinengestell entbehrlich wird. Das Dokument DE-A-19743076 offenbart ein Verfahren zum thermischen Entgraten von Werkstücken in einer verschliessbaren Entgratungskammer durch Zünden einer in die Entgratungskammer eingebrachten, brennbaren Prozessgasfüllung, wobei die nach dem Verschliessen der Entgratungskammer in dieser vorhandene Luft zumindest beim Einleiten der brennbaren Prozessgasfüllung zumindest teilweise entfernt wird.

### ZUSAMMENFASSUNG DER ERFINDUNG

Der vorliegenden Erfindung liegt der Gedanke zugrunde, angesichts der oben beschriebenen Problematik die Fertigungsqualität und damit unter anderem auch die Passgenauigkeit von Polymerkomponenten zu verbessern, um dadurch die Anzahl von Polymerpartikeln zu reduzieren. Ziel hiervon ist, die Wahrscheinlichkeit des Auftretens einer Osteolyse zu senken.

Die vorliegende Erfindung erreicht dies mit dem im unabhängigen Anspruch 1 definierten Verfahren. Die dazugehörigen abhängigen Ansprüche definieren dabei bevorzugte Ausführungsformen.

Das von der Erfindung bereitgestellte Verfahren zur Bearbeitung eines Polymerwerkstücks für einen Einsatz mit und insbesondere in einem Gelenkimplantat umfasst ein Platzieren des Polymerwerkstücks in einer Explosionskammer, ein Einführen eines brennbaren Gasgemischs in die Explosionskammer und ein Entzünden des brennbaren Gasgemischs, wobei durch Entzünden des Gasgemischs in der Explosionskammer eine Temperatur erzeugt wird, die oberhalb des Schmelzpunktes eines Polymers von dem Polymerwerkstück liegt.

Die durch das Entzünden des Gasgemischs hervorgerufene Explosion bzw. schlagartige Verbrennung führt dazu, dass etwaige hervorstehende Polymerabschnitte durch Verbrennung oder Verdampfung entfernt werden. Insbesondere werden durch das Verfahren herstellungsbedingte Unstetigkeiten bzw. Abweichungen von der herzustellenden Geometrie entfernt, wie zum Beispiel hervorstehende Grate oder nicht abgelöste Späne. Da nach dem Entfernen der herstellungsbedingten Unstetigkeiten keine Materialreste oder Stoffe an dem Polymerwerkstück verbleiben, können die oben genannten negativen Effekte nach der Implantation aufgrund dieser nicht mehr auftreten. Dabei ermöglicht das Verfahren die Entfernung dieser Unstetigkeiten, ohne dabei die Biokompatibilität des Polymerwerkstoffs herabzusetzen.

Da die Verbrennung explosionsartig erfolgt, ist dieses Verfahren im Vergleich zu den üblichen Verfahren, wie zum Beispiel einer manuellen Nachbearbeitung, wesentlich schneller. Zudem spielt auch eine möglicherweise komplexe Geometrie des Polymerwerkstücks keine Rolle. Selbst schwer zugängliche Abschnitte auf der Oberfläche des Polymerwerkstücks werden durch dieses Verfahren erreicht und von Unstetigkeiten gesäubert. Das Entfernen der Unstetigkeiten in Übereinstimmung mit der vorliegenden Erfindung verhindert, dass diese bei Implantation in den Körper des Patienten gelangen. Letzteres kann vor allem beim manuellen Entgraten auftreten.

Im Gegensatz zu dem entfernten Material kommt es bei dem restlichen Material zu keiner nachteiligen Veränderung. Die für die Fertigung des Polymerwerkstücks vorgesehene Geometrie und deren Abmessungen bleiben im Wesentlichen unverändert. Die hierdurch erreichte Passgenauigkeit vermeidet Relativbewegungen zwischen der Verankerungskomponente und dem Polymerwerkstück und beugt damit einem Ablösen von Polymerpartikeln vor.

Das Polymerwerkstück weist bevorzugt nur ein Polymer auf, kann aber auch durch einen Polymerverbund oder eine Polymermischung ausgebildet sein. Das Polymerwerkstück bildet vorzugsweise mindestens einen Abschnitt der Gelenkfläche des Gelenkimplantats aus. An dem Polymerwerkstück ist bevorzugt mindestens ein Element für eine Verbindung mit einer Verankerungskomponente vorgesehen, mit der das Polymerwerkstück an Knochengewebe verankerbar ist.

Bei einer weiteren bevorzugten Ausführungsform weist das Polymerwerkstück einen Thermoplast, bevorzugt Polyethylen und noch bevorzugter UHMWPE auf.

Das Verfahren ist besonders bei Thermoplasten vorteilhaft anwendbar, da diese Werkstoffe sich chemisch durch die bei der Explosion entstehenden Wärme nicht verändern. Hinzu kommt, dass nicht nur die oben genannten Unstetigkeiten entfernt werden, sondern auch Abweichungen in der Werkstückoberfläche geglättet werden.

Ein für das Verfahren besonders geeigneter Thermoplast ist Polyethylen. Polyethylen hat sich als Werkstoff in künstlichen Gelenken bewährt. Dies ist insbesondere für UHMWPE bei Gelenkflächen der Fall. Zudem konnte festgestellt werden, dass die Bearbeitung des Polymerwerkstücks trotz der zur Anwendung kommenden hohen Temperaturen keine feststellbare Veränderung des Werkstoffs hervorruft, welche die Biokompatibilität oder Funktionalität und damit den Einsatz im Körper beeinträchtigen könnten.

Bei einer weiteren besonders bevorzugten Ausführungsform liegt die Temperatur in einem Bereich von 1500°C bis 2800°C und bevorzugt in einem Bereich von 2000°C bis 2500°C.

Die genannten für ein Polymerwerkstück hohen Temperaturbereiche haben sich als besonders zuverlässig für die Entfernung der Unstetigkeiten erwiesen. Auch wird durch sie auf der Oberfläche der Werkstücke der genannte Glättungseffekt erreicht, bei dem herstellungsbedingte Unebenheiten geglättet werden, ohne dabei die bei der Herstellung vorgesehenen Abmessungen des Werkstücks zu beeinflussen. Mit anderen Worten ist es aufgrund des Verfahrens nicht notwendig, bei der Fertigung eine Materialzugabe ähnlich wie bei Gusstücken vorzusehen.

Bei einer weiteren bevorzugten Ausführungsform wird das zur Explosion zu bringende Gasgemisch bis zu einem Druck von 1,5 bis 2,1 bar und bevorzugt von 1,7 bis 1,9 bar eingeführt.

Das Gasgemisch wird solange in die Explosionskammer eingeführt, bis die genannten Druckbereiche erreicht werden. Die so zur Verfügung gestellte Gasmenge reicht aus, um durch eine einmalige explosionsartige Verbrennung die Temperaturen zu erreichen, mit denen die Nachbearbeitung des Polymerwerkstücks stattfinden kann. Mit anderen Worten muss durch diese Drücke nur einmalig vor der Explosion Gasgemisch zugeführt werden. Ein Nachführen von Gas ist nicht notwendig.

Bei einer weiteren Ausführungsform wird das Polymerwerkstück spanend vorbearbeitet.

Das Verfahren ist insbesondere bei spanend vorbearbeiteten Werkstücken vorteilhaft, da beim spanenden Bearbeiten vermehrt Unstetigkeiten wie Grate und nicht vollständig abgelöste Späne entstehen, die üblicherweise mit hohem Kostenaufwand manuell per Hand entfernt werden müssen. Dabei kommt bei Gelenkkomponenten erschwerend hinzu, dass diese häufig eine komplexe Geometrie aufweisen, die einerseits durch die Gelenkfläche hervorgerufen wird und andererseits funktionell notwendig sind, wie beispielsweise durch die Verbindung mit einer Verankerungskomponente. Weiterer Vorteil des Verfahrens ist in Bezug auf spanende Fertigungsverfahren, dass ein Schlichten der Werkstücke zumindest teilweise nicht mehr notwendig ist, da das Verfahren die durch das Schruppen häufiger verursachten Unstetigkeiten ähnlich gut entfernt.

Bei einer weiteren besonders bevorzugten Ausführungsform des Verfahrens wird die durch die Explosion des Gasgemischs hervorgerufene Temperatur von mindestens 1500°C, bevorzugt von mindestens 2000°C, über einen Zeitraum von 1 ms bis zu 10 ms, bevorzugt 1 ms bis 5 ms und noch bevorzugter 1 ms bis 2 ms gehalten.

Diese Kombinationen aus Mindesttemperatur und Zeitdauer, über welche die Temperatur gehalten wird, sorgt für die Entfernung der durch die Formgebung verbliebenen Unstetigkeiten. Dabei reichen die kürzeren Zeiträume bereits aus, um zumindest frei stehende Unstetigkeiten zu verbrennen oder zu verdampfen. Je länger der Zeitraum ist, desto stärker ist der zusätzliche Glättungseffekt ausgeprägt.

Bei einer besonders bevorzugten Ausführungsform der Erfindung weist das Gasgemisch Sauerstoff und Methan auf.

Dieses Gasgemisch erzeugt die notwendige Temperatur zum Entfernen etwaiger Unstetigkeiten an dem Werkstück auf zuverlässige und kontrollierbare Weise. Zudem stellt es eine saubere Verbrennung sicher, sodass keine Rückstände auf dem Werkstück verbleiben, die anderweitig entfernt werden müssten, um die Biokompatibilität des Polymerwerkstücks zu erhalten. Das Gasgemisch wird bevorzugt vorgemischt, um eine homogene Verteilung der Bestandteile des Gasgemischs sicherzustellen.

Des Weiteren stellt die vorliegende Erfindung ein Gelenkimplantat mit einer Polymerkomponente bereit, wobei die Polymerkomponente mit dem oben beschriebenen Verfahren hergestellt worden ist und die Polymerkomponente Teil eines Hüftgelenkersatzes, eines Kniegelenkersatzes, insbesondere eines Tibiaplateaus, eines Schultergelenkersatzes, eines Sprunggelenkersatzes, eines Ellengelenkersatzes, eines Fingergelenkersatzes oder einer Megaprothese ist.

Die vorliegende Erfindung stellt auch ein Instrument für eine Implantation eines Gelenkersatzes bereits, das eine Komponente aus einem sterilisierbaren Thermoplast aufweist, die mit dem oben beschriebenen Verfahren hergestellt worden ist.

Zusammenfassend leistet das Verfahren eine Bearbeitung bzw. Nachbearbeitung des Werkstücks auf eine äußerst effiziente Weise, und zwar nicht nur in Bezug auf die Herstellungskosten. Durch die hohe Zuverlässigkeit bei der Entfernung von Unstetigkeiten auf der Werkstückoberfläche wird auch einem Ablösen dieser Unstetigkeiten nach Implantation erfolgreich vorgebeugt und damit den oben beschriebenen negativen Folgen von in Gewebe eingelagerten oder an Gewebe angelagerten Polymerpartikeln.

### KURZE BESCHREIBUNG DER FIGUREN

Die folgenden Figuren veranschaulichen die nachfolgende ausführliche Erläuterung bevorzugter Ausführungsformen der vorliegenden Erfindung, wobei
Figur 1 ein Polymerwerkstück für einen Einsatz in einem Gelenkimplantat zeigt,
Figur 2 einen Abschnitt des Polymerwerkstücks aus Figur 1 nach einer spanenden Bearbeitung zeigt,
Figur 3 einen mit dem Abschnitt aus Figur 2 vergleichbaren Abschnitt nach einer manuellen Entgratung per Hand zeigt,
Figur 4 einen mit dem Abschnitt aus Figur 2 vergleichbaren Abschnitt nach einer Bearbeitung des Werkstücks mit dem erfindungsgemäßen Verfahren zeigt,
Figur 5 eine Aufnahme mit einem Rasterelektronenmikroskop zeigt, wobei Figur 5a) die Oberfläche des Werkstücks in einem Kantenbereich nach einer manuellen Entgratung per Hand veranschaulicht und Figur 5b) die Oberfläche des Werkstücks in einem vergleichbaren Kantenbereich nach Anwendung des erfindungsgemäßen Verfahrens veranschaulicht,
Figur 6 eine Aufnahme mit einem Rasterelektronenmikroskop in einer im Vergleich zu Figur 5 höheren Auflösung zeigt, wobei Figur 6a) die Oberfläche des Werkstücks in einem Kantenbereich nach einer manuellen Entgratung per Hand veranschaulicht und Figur 6b) die Oberfläche des Werkstücks in einem vergleichbaren Kantenbereich nach Anwendung des erfindungsgemäßen Verfahrens veranschaulicht, und
Figur 7 Werkstücke in einer Halterung vor Ausführung des Explosionsentgratens zeigt.

### AUSFÜHRLICHE BESCHREIBUNG BEVORZUGTER AUSFÜHRUNGSFORMEN

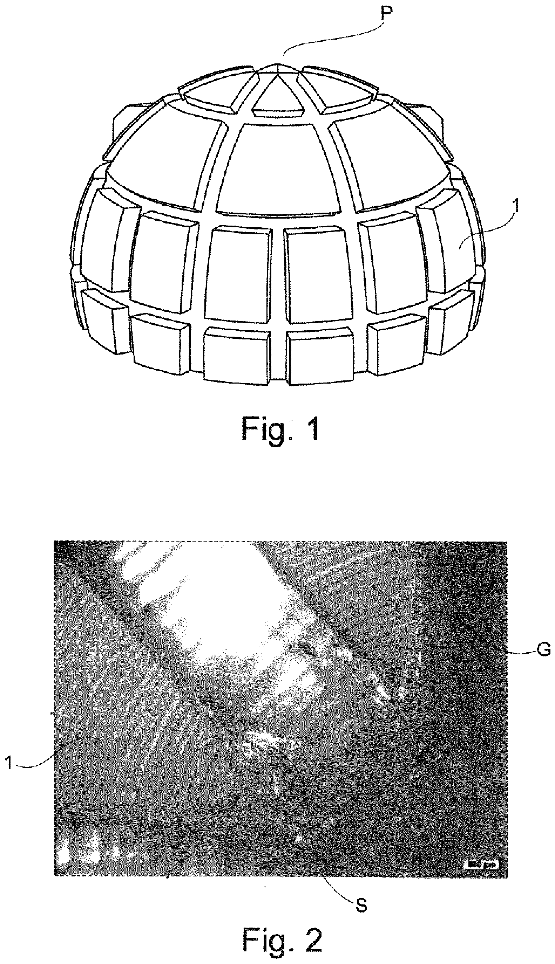

Figur 1 zeigt ein Polymerwerkstück 1, das für einen Einsatz in einem Gelenkimplantat vorgesehen ist. Bei dem gezeigten Polymerwerkstück 1 handelt es sich um einen Hüftgelenkeinsatz aus UHMWPE, der in eine metallene Hüftpfanne einsetzbar ist, die wiederum für eine Implantation im Acetabulum eines Patienten vorgesehen ist. Anders ausgedrückt wird der in Figur 1 veranschaulichte Gelenkeinsatz mit einer nicht gezeigten Hüftpfanne aus einem Polymerwerkstoff im Knochengewebe verankert.

Anhand der Figur 1 wird auch die Komplexität der Außengeometrie deutlich, die ein Gelenkeinsatz aufweisen kann. Dabei ist der in Figur 1 gezeigte Hüftgelenkeinsatz lediglich ein Beispiel für die vielseitigen Anwendungsmöglichkeiten des Verfahrens bei Gelenkimplantaten. Es kann bei unterschiedlichsten Gelenken, wie zum Beispiel Hüftpfannen bzw. -einsätzen, Tibiaplateaus von Kniegelenken, Kniegelenkeinsätzen, Schulterprothesenkomponenten, Sprunggelenkskomponenten, Ellengelenkskomponenten, Megaprothesenkomponenten, sowie den Instrumenten eingesetzt werden, die aus sterilisierbaren Thermoplasten hergestellt werden.

Aufgrund der genauen und zuverlässigen Entfernung der Unstetigkeiten, die im Wesentlichen ohne Materialabtrag der Sollgeometrie erfolgt, ist das Verfahren auch besonders vorteilhaft bei kleinen Gelenkkomponenten einsetzbar, wie zum Beispiel Fingergelenken, wie dem Daumensattelgelenk. Gerade bei diesen vergleichsweise kleinen Gelenken und damit kleinen Polymerwerkstücken 1 führt das Entgraten per Hand zu einer relativ starken Veränderung der Werkstückgeometrie.

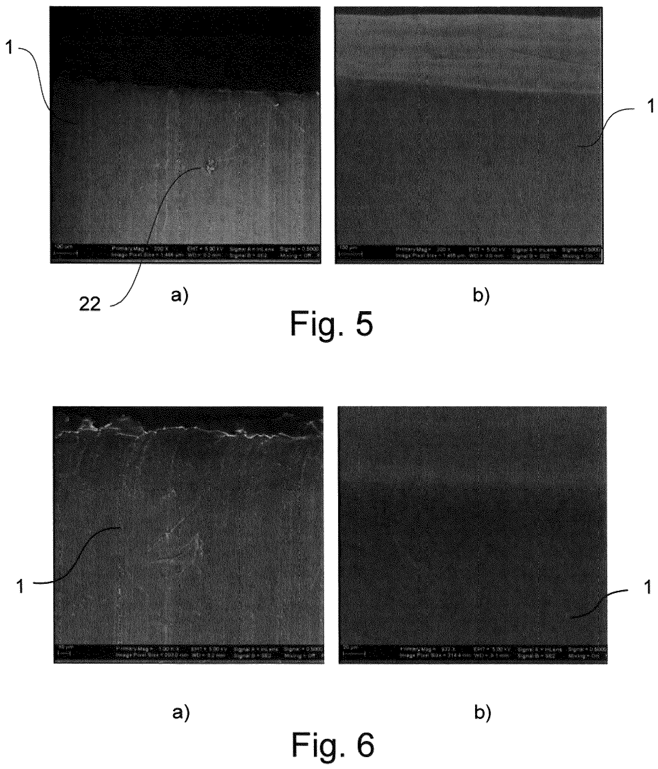

Unter Unstetigkeiten werden im Rahmen dieser Erfindung Überstände verstanden, die auf die Fertigung des Polymerwerkstücks 1 zurückzuführen sind. Hierzu gehören Grate G und Späne S, die im Allgemeinen durch Schneidprozesse hervorgerufen werden, aber auch in die Werkstückoberfläche eingedrückte oder angedrückte Polymerpartikel 22 (vergleiche Figur 5a). Weiterhin ist es auch möglich, durch Schneidprozesse eingebrachte Unstetigkeiten in der Oberfläche zu verringern.

Für den Einsatz in einem Gelenkersatz vorgesehene Werkstücke 1 werden bevorzugt durch Spritzguss urgeformt. Folglich wird als Polymerwerkstoff ein Thermoplast bevorzugt, der zudem die oben bereits genannten Vorteile im Zusammenhang mit dem Verfahren aufweist. Im Regelfall kommen auch spanende Verarbeitungsverfahren zur Anwendung, sei es um eine Geometrie aus dem Vollen zu drehen oder zu fräsen oder nach dem Urformen stehen gebliebene Überstände, wie zum Beispiel Gussansätze, zu entfernen.

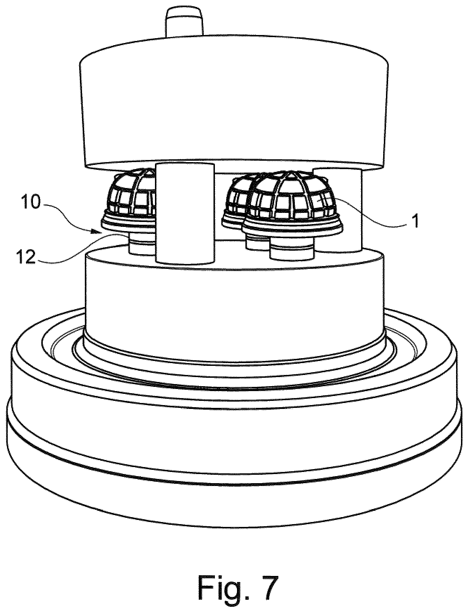

Ein so vorgefertigtes und wie in Figur 1 beispielhaft veranschaulichtes Polymerwerkstück 1 wird anschließend für die Anwendung des Verfahrens in einer Explosionskammer oder einem Explosionskammereinsatz 10 an einer Halterung 12 fixiert (vgl. Figur 7). Bei dem in der Figur 7 dargestellten Explosionskammereinsatz 10 können drei Polymerwerkstücke 1 gleichzeitig behandelt werden. Die Explosionskammer wird durch Einsetzen des Explosionskammereinsatzes 10 in eine entsprechende Öffnung ausgebildet. Das dadurch festgelegte freie Volumen der Explosionskammer, welches mit dem Gasgemisch gefüllt wird, beträgt in etwa das 10-fache bis 30-fache, bevorzugt 15-fache bis 25-fache des Volumens der Polymerwerkstücke 1.

Nach der Platzierung in der Explosionskammer wird diese verschlossen und es wird das brennbare bevorzugt vorgemischte Gasgemisch, wie zum Beispiel das oben genannte, eingeführt.

Anschließend wird das Gasgemisch entzündet, sodass es zu einer explosionsartigen Verbrennung, ähnlich wie bei einer Kraftbrennmaschine, kommt. Dabei wird der Verbrennungsprozess insbesondere durch das Zuführen einer entsprechenden Menge des Gasgemischs so gesteuert, dass in der Explosionskammer Temperaturen von 1500°C bis 2800°C und bevorzugt von 2000°C bis 2500°C erreicht werden.

Der Zeitraum, über den Temperaturen des genannten Temperaturbereichs erreicht werden, ist so gewählt, dass vorhandene Unstetigkeiten durch Verbrennung oder Verdampfung entfernt werden. Die hierfür notwendige Zeitdauer befindet sich im Allgemeinen im Millisekundenbereich und insbesondere in einem Bereich von 1 ms bis zu 10 ms, bevorzugt 1 ms bis 5 ms und noch bevorzugter 1 bis 2 ms. Dabei reicht ein sehr kurzer Zeitraum, wie beispielsweise der letztgenannte, aus, um vorhandene Unstetigkeiten im Wesentlichen zu entfernen. Die genannten längeren Zeiträume führen zusätzlich zu einem verstärkten Glättungseffekt auf der Oberfläche des Werkstücks 1.

Der durch die Verbrennung entstehende Überdruck wird kontrolliert aus der Explosionskammer abgeführt. Der gesamte Vorgang des Explosionsentgratens nimmt nur in etwa 1 Minute in Anspruch. Mit anderen Worten vergeht von Zündung zu Zündung in etwa 1 Minute, wobei bei jeder Zündung mehrere Werkstücke 1 gleichzeitig verarbeitet werden können, wie durch Figur 7 veranschaulicht.

Anhand der Figuren 2 bis 6 werden nachfolgend die durch das Verfahren erreichten Vorteile beispielhaft anhand der Bearbeitung eines wie in der Figur 1 gezeigten Polymerwerkstücks 1 veranschaulicht.

Figur 2 zeigt den Polabschnitt P aus Figur 1 in vergrößerter Darstellung nach erfolgter spanender Bearbeitung, mit der die in Figur 1 veranschaulichte komplexe Geometrie hergestellt worden ist. In Figur 2 sind die durch die spanende Bearbeitung entstandenen Grate G und Späne S, welche sich nicht vom Werkstück 1 gelöst haben, zu erkennen.

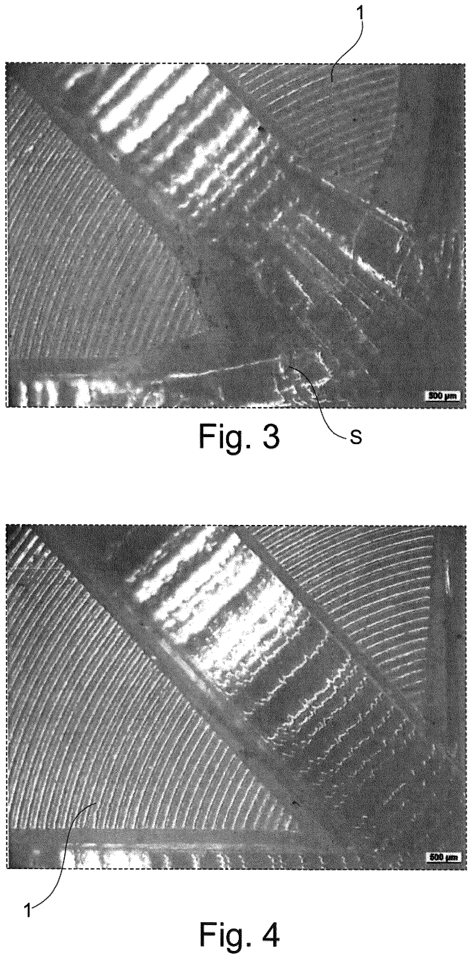

Ein für derartige Werkstücke 1 übliches Bearbeitungsverfahren ist das manuelle Entgraten, das vorwiegend durch Handarbeit erfolgt. Das Ergebnis einer derartigen Entgratung per Hand ist in Figur 3 veranschaulicht, die einen dem in Figur 2 gezeigten Abschnitt entsprechenden Abschnitt bei gleicher Vergrößerung zeigt.

Bei der Entgratung handelt es sich ebenfalls um ein spanendes Verfahren. Mit anderen Worten besteht durch das Entgraten per Hand die Möglichkeit, dass Späne und Grate nicht nur zurückbleiben, sondern auch neu entstehen. Einer dieser Gründe hat dazu geführt, dass in Figur 3 nach wie vor Span S vorhanden ist.

Besonders deutlich tritt in Figur 3 zudem die bei der manuellen Entgratung auftretende Veränderung der Werkstückgeometrie hervor. So sind die dreiecks- bzw. tortenstückförmigen Spitzen auf der Polseite P des Polymerwerkstücks 1 der Figur 1 aufgrund der manuellen Nachbearbeitung zurückgesetzt und abgestumpft. Durch diese lokale Verkleinerung der Geometrie erhöht sich die Wahrscheinlichkeit, dass zwischen dem gezeigten Polymerwerkstück 1 und der zugehörigen Verankerungskomponente, d. h. in diesem Fall einer Hüftpfanne, eine Relativbewegung möglich ist. Solche Bewegungen im Mikrometerbereich können zusätzlich Polymerpartikel herauslösen. Auch dies begünstigt das Auftreten einer Osteolyse im Bereich des Knochengewebes, das in der Umgebung des künstlichen Gelenkersatzes, d. h. der Verankerungskomponente, liegt. Dies gilt vor allem für den dem Gewebe zugewandten Schnittstellenbereich zwischen dem Polymerwerkstück 1 und der damit verbundenen Verankerungskomponente.

Im Gegensatz zur Figur 3 wurde als Bearbeitungsverfahren bei dem in Figur 4 gezeigten Abschnitt eines Polymerwerkstücks 1 das erfindungsgemäße Verfahren angewandt. Durch diesen Ausschnitt, der wiederum dem in Figur 2 gezeigten Ausschnitt des unbearbeiteten Polymerwerkstücks 1 entspricht, ist deutlich erkennbar, dass die nach dem erfindungsgemäßen Verfahren verbleibende Werkstückgeometrie der idealen, das heißt, der durch die Konstruktion vorgesehenen Geometrie, wesentlich näher kommt. Folglich kann bei Abschnitten für eine Verbindung zwischen dem Polymerwerkstück 1 und der Verankerungskomponente eine höhere Passgenauigkeit erreicht, und damit den oben erwähnten Mikrobewegungen vorgebeugt werden.

Die Figuren 5 und 6 zeigen einen vergrößerten Abschnitt eines Kantenbereichs des Polymerwerkstücks 1 aus Figur 1. Dabei zeigen die Figuren 6a und 6b einen Abschnitt wie die Figuren 5a und 5b, jedoch in nochmals vergrößerter Ansicht. Die Figuren 5a und 6a veranschaulichen das Ergebnis, dass durch eine Handentgratung erreicht wird, während die Figuren 5b und 6b das durch das erfindungsgemäße Verfahren erreichte Bearbeitungsergebnis zeigen.

An dem in den Figuren 5a und 6a gezeigten Werkstück 1 wurde eine manuelle Entgratung durchgeführt, bei der jedoch aufgrund des Entgratungsverfahrens und der Eigenschaften des Polymerwerkstoffs lediglich die in Figur 2 zu erkennenden makroskopischen Unstetigkeiten entfernt werden konnten. Anhand der in den Figuren 5a und 6a gezeigten rasterelektronenmikroskopischen Aufnahmen wird jedoch deutlich, dass beim Entgraten die verbleibenden Grate oder Späne nicht vollständig entfernt werden konnten. Auch entstehen durch das Entgraten, welches selbst ein Schneidverfahren ist, ein erneuter, wenn auch kleinerer Grat. Dieser ist im oberen Bereich der Figur 5a und noch einmal vergrößert in dem oberen Bereich der Figur 6a deutlich erkennbar veranschaulicht.

Weiterhin wird in der Figur 5a deutlich, dass auf der Oberfläche bedingt durch das Entgratungsverfahren Polymerpartikel 22 in die Werkstückoberfläche eingepresst werden. Bei diesen besteht die Gefahr, dass sie sich nach der Implantation durch mechanische Belastung lösen und die oben genannten Nachteile hervorrufen können.

Die in den Figuren 5b und 6b gezeigten Abschnitte eines Polymerwerkstücks 1, die eine durch das Verfahren erzeugte Werkstückoberfläche zeigt, hat dagegen ein wesentlich gleichförmigeres Erscheinungsbild. In den Figuren 5b und 6b können keine zu den Figuren 5a und 6a vergleichbare Unstetigkeiten in der Werkstückoberfläche festgestellt werden. Stattdessen hat die gezeigte Oberfläche ein nahezu glattes Erscheinungsbild, welches durch den kurzzeitigen hohen Temperaturanstieg in der Explosionskammer während des Verfahrens hervorgerufen wird. Auch hierdurch wird einem späteren Ausbrechen oder Ablösen von Polymerpartikeln 22 aus dem Polymerwerkstück 1 bei implantiertem künstlichen Gelenk vorgebeugt.

Insbesondere wird einem Ausbrechen von Polymerpartikeln 22 gerade im Randbereich einer Gelenkfläche vorgebeugt. Gerade in diesem Bereich, in dem das Polymerwerkstück 1 im implantierten Zustand durch den Flächenkontakt mit dem Gelenkpartner stark belastet wird, ist die Gefahr des Ausbrechens von Polymerpartikeln besonders hoch, kann aber durch Anwendung des vorliegenden Verfahrens deutlich gesenkt werden.

Insgesamt werden somit nicht nur Kostenvorteile bedingt durch das Wegfallen der aufwendigen Entgratung per Hand und eine deutliche Beschleunigung der Bearbeitung, sondern auch qualitative Vorteile durch die gestiegene Genauigkeit und umfassende Bearbeitung der Werkstückoberfläche erreicht.

### BEZUGSZEICHEN

- 1: Polymerwerkstück
- 10: Explosionskammereinsatz
- 12: Halterung für Werkstück
- 22: Polymerpartikel

- P: Polseite
- G: Grat
- S: Span

## Patentansprüche

1. Verfahren zur Bearbeitung eines Polymerwerkstücks (1) einer Gelenkersatzkomponente, das die Schritte umfasst:
- Platzieren des Polymerwerkstücks (1) in einer Explosionskammer,
- Schließen der Explosionskammer,
- Einführen eines brennbaren Gasgemischs in die Explosionskammer,
- Entzünden des brennbaren Gasgemischs,
wobei durch Entzünden des Gasgemischs in der Explosionskammer eine Temperatur erzeugt wird, die oberhalb des Schmelzpunkts eines Polymers von dem Polymerwerkstück (1) liegt.

2. Verfahren nach Anspruch 1, bei dem das Polymerwerkstück (1) einen Thermoplasten, bevorzugt Polyethylen und noch bevorzugter UHMWPE aufweist.

3. Verfahren nach Anspruch 1 oder 2, bei dem die durch die Explosion erreichte Temperatur in einem Bereich von 1500°C bis 2800°C und bevorzugt in einem Bereich von 2000°C bis 2500°C liegt.

4. Verfahren nach einem der vorstehenden Ansprüche, bei dem das zur Explosion zu bringende Gasgemisch bis zu einem Druck von 1,5 bis 2,1 bar und bevorzugt von 1,7 bis 1,9 bar eingeführt wird.

5. Verfahren nach einem der vorstehenden Ansprüche, bei dem das Polymerwerkstück (1) spanend vorbearbeitet wird.

6. Verfahren nach einem der vorstehenden Ansprüche, bei dem die durch die Explosion des Gasgemischs hervorgerufene Temperatur von mindestens 1500°C, bevorzugt von mindestens 2000°C, über einen Zeitraum von 1 ms bis zu 10 ms, bevorzugt 1 ms bis 5 ms und noch bevorzugter 1 ms bis 2 ms gehalten wird.

7. Verfahren nach einem der vorstehenden Ansprüche, bei dem das Gasgemisch Sauerstoff und Methan aufweist.

8. Gelenkimplantat mit einer Polymerkomponente, wobei die Polymerkomponente mit dem Verfahren nach einem der vorstehenden Ansprüche hergestellt worden ist und die Polymerkomponente Teil eines Hüftgelenkersatzes, eines Kniegelenkersatzes, insbesondere eines Tibiaplateaus, eines Schultergelenkersatzes, eines Sprunggelenkersatzes, eines Ellengelenkersatzes, eines Fingergelenkersatzes oder einer Megaprothese ist.

## Claims

1. Method for treating a polymer workpiece (1) of a joint-replacement component, comprising the steps of:
- placing the polymer workpiece (1) in an explosion chamber,
- closing the explosion chamber,
- introducing a combustible gas mixture into the explosion chamber,
- igniting the combustible gas mixture,
wherein ignition of the gas mixture in the explosion chamber generates a temperature which lies above the melting point of a polymer of the polymer workpiece (1).

2. Method according to Claim 1, where the polymer workpiece (1) comprises a thermoplastic, preferably polyethylene, and more preferably still UHMWPE.

3. Method according to Claim 1 or 2, where the temperature reached by the explosion is in a range from 1500°C to 2800°C and preferably in a range from 2000°C to 2500°C.

4. Method according to any of the preceding claims, where the gas mixture to be exploded is introduced at a pressure of 1.5 to 2.1 bar and preferably of 1.7 to 1.9 bar.

5. Method according to any of the preceding claims, where the polymer workpiece (1) is cuttingly pretreated.

6. Method according to any of the preceding claims, where the temperature brought about by the explosion of the gas mixture of at least 1500°C, preferably of at least 2000°C, is maintained over a period of 1 ms to 10 ms, preferably 1 ms to 5 ms and more preferably still 1 ms to 2 ms.

7. Method according to any of the preceding claims, where the gas mixture comprises oxygen and methane.

8. Joint-implant having a polymer component, wherein the polymer component has been produced by the method according to any of the preceding claims and the polymer component is part of a hip-joint replacement, a knee-joint replacement, more particularly a tibia plateau, a shoulder-joint replacement, an ankle-joint replacement, an elbow-joint replacement, a finger-joint replacement or a megaprosthesis.

## Revendications

1. Procédé de traitement d'une pièce en polymère (1) d'un composant de remplacement d'articulation, ledit procédé comprenant les étapes suivantes :
- placer la pièce en polymère (1) dans une chambre d'explosion,
- fermer la chambre d'explosion,
- introduire un mélange gazeux combustible dans la chambre d'explosion,
- enflammer le mélange gazeux combustible,
une température supérieure au point de fusion d'un polymère de la pièce en polymère (1) étant générée par inflammation du mélange gazeux dans la chambre d'explosion.

2. Procédé selon la revendication 1, dans lequel la pièce en polymère (1) comprend un thermoplastique, de préférence du polyéthylène et plus préférablement du UHMWPE.

3. Procédé selon la revendication 1 ou 2, dans lequel la température atteinte lors de l'explosion est dans une gamme allant de 1500 °C à 2800 °C et de préférence dans une gamme allant de 2000 °C à 2500 °C.

4. Procédé selon l'une des revendications précédentes, dans lequel le mélange gazeux à faire exploser est introduit jusqu'à atteindre une pression de 1,5 à 2,1 bar et de préférence de 1,7 à 1,9 bar.

5. Procédé selon l'une des revendications précédentes, dans lequel la pièce en polymère (1) est préalablement usinée.

6. Procédé selon l'une des revendications précédentes, dans lequel la température provoquée par l'explosion du mélange gazeux d'au moins 1500 °C, de préférence d'au moins 2000 °C, est maintenue pendant une durée de 1 ms à 10 ms, de préférence de 1 ms à 5 ms et plus préférablement de 1 ms à 2 ms.

7. Procédé selon l'une des revendications précédentes, dans lequel le mélange gazeux comprend de l'oxygène et du méthane.

8. Implant articulaire comprenant un composant en polymère, le composant en polymère ayant été réalisé à l'aide du procédé selon l'une des revendications précédentes et le composant en polymère faisant partie d'une prothèse articulaire de la hanche, d'une prothèse articulaire du genou, notamment d'un plateau tibial, d'une prothèse articulaire de l'épaule, d'une prothèse articulaire de la cheville, d'une prothèse articulaire du coude, d'une prothèse articulaire des doigts ou d'une mégaprothèse.
